# EUROPEAN PATENT APPLICATION

(11) **EP 2 881 387 A1**
(43) Date of publication of application: **10.06.2015**
(21) Application number: 13196273.0
(22) Date of filing: 09.12.2013
(51) Int. Cl.: C07D 231/28, C07D 401/04, A01N 43/56

(54) **Pyrazolone compounds having herbicidal activity**

(71) Applicant: BASF SE, 67056 Ludwigshafen (DE)
(72) Inventor: Parra Rapado, Liliana, Dr., 77654 Offenburg (DE); Pasternak, Maciej, Dr., 67067 Ludwigshafen (DE); Kreuz, Klaus, Dr., 79211 Denzlingen (DE); Hutzler, Johannes, Dr., 67165 Waldsee (DE); Besong, Gilbert, Dr., 67098 Bad Dürkheim (DE)

(57) **Abstract**

The present invention provides a pyrazolone compound of the formula I 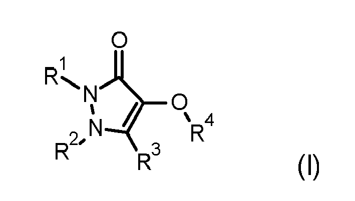 or an agriculturally suitable salt or N-oxide thereof, wherein the variables in the formula I are defined as in the description. Pyrazolone compounds of formula I are useful as herbicides.

## Description

The present invention relates to pyrazolone compounds of the general formula (I) defined below and to their use as herbicides. Moreover, the invention relates to compositions for crop protection and to a method for controlling unwanted vegetation.

WO 97/13757, WO 2006/071730 and US 2007/0049574 A1 describe certain pyrazolone derivatives which are useful as pharmaceuticals.

WO 2011/051958 describes certain pyrazolones and their use as fungicides.

WO 2013/050421 and WO 2013/083774 both describe certain herbicidal pyridazinone derivatives.

However, the herbicidal properties of these known compounds with regard to the harmful plants are not always entirely satisfactory.

It is therefore an object of the present invention to provide alternative herbicidally active compounds. To be provided are in particular compounds which have high herbicidal activity, in particular even at low application rates, and which are sufficiently compatible with crop plants for commercial utilization.

These and further objects are achieved by pyrazolone compounds of formula (I), defined below, and by their agriculturally suitable salts or N-oxides.

Accordingly, the present invention provides pyrazolone compound of the formula I 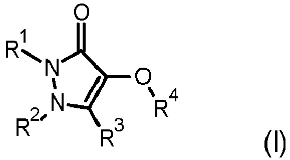 or an agriculturally suitable salt or N-oxide thereof,
wherein
R¹ and R² are each independently selected from the group consisting of hydrogen, C₁-C₆-alkyl, C₃-C₆-cycloalkyl, C₁-C₆-haloalkyl, C₁-C₆-alkoxy-C₁-C₃-alkyl, C₃-C₆-cycloalkyl-C₁-C₃-alkyl-, oxetanyl and tetrahydropyranyl;
R³ is a group represented by the formula A1 or A2 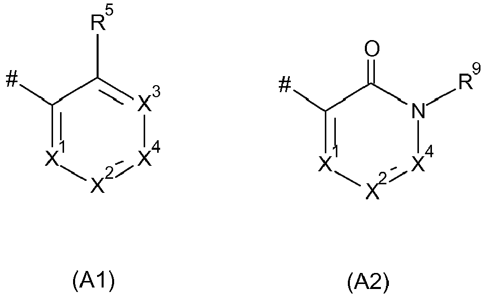
wherein in each of the formulae A1 and A2
# denotes the bonding site to the remainder of the formula (I);
X¹ is N or CR7;
X² is N or CR⁸;
X³ is N or CR⁹;
X⁴ is N or CR⁶;
R⁴ is selected from the group consisting of hydrogen, C₁-C₆-alkylcarbonyl-, arylcarbonyl-, C₁-C₆alkoxycarbonyl-, C₁-C₆-alkoxycarbonyl-C₁-C₃-alkoxy-, C₁-C₆-alkylcarbonyl-C₁-C₃-alkoxy-, C₁-C₆-alkyl-S(O)ₙ-, C₁-C₆-alkyl-S(O)ₙcarbonyl- and aryl-S(O)ₙ-, wherein said aryl groups are unsubstituted or substituted by one to seven R¹⁰;
R⁵ is selected from the group consisting of hydroxyl, halogen, C₁-C₆-alkyl, C₃-C₆-cycloalkyl, C₁-C₆-haloalkyl, C₁-C₆-alkoxy-, C₃-C₆-cycloalkyl-C₁-C₃-alkyl-, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₃-alkyl-, C₁-C₆-haloalkoxy-, C₁-C₆-haloalkoxy-C₁-C₃-alkyl-, C₁-C₆-alkyl-S(O)ₙ-, C₁-C₆-haloalkyl-S(O)ₙ-, aryl-S(O)ₙ-, heterocyclyl, heterocyclyl-S(O)ₙ-, heterocyclyloxy-, heterocyclyl-C₁-C₃-alkoxy-C₁-C₃-alkyl-, C₁-C₃-alkoxycarbonyl-, C₁-C₃-alkoxycarbonyl-C₁-C₃-alkoxy-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, cyano and nitro, wherein said heterocyclyls are five or six membered heterocyclyls containing from one to three heteroatoms each independently selected from the group consisting of oxygen, nitrogen and sulphur, and wherein the aryl or heterocyclyl components may be unsubstituted or substituted by one to four substituents each independently selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, cyano and nitro;
R⁶ is selected from the group consisting of hydrogen, hydroxyl, halogen, C₁-C₆-alkyl, C₃-C₆-cycloalkyl, C₁-C₆-haloalkyl, C₂-C₆-alkenyl, C₂-C₆-haloalkenyl, C₂-C₆-alkynyl, C₁-C₆-alkoxy-, C₂-C₆-alkenyloxy-, C₃-C₆-cycloalkyl-C₁-C₃-alkyl-, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₃-alkyl-, C₁-C₆-haloalkoxy-, C₁-C₆-haloalkoxy-C₁-C₃-alkyl-, C₁-C₆-alkyl-S(O)ₙ-, C₁-C₆-haloalkyl-S(O)ₙ-, aryl, aryl-S(O)ₙ-, heterocyclyl, heterocyclyl-S(O)ₙ-, aryloxy-, aryl-C₂-C₆-alkyl-, aryl-C₁-C₆-alkoxy-, heterocyclyloxy-, heterocyclyl-C₁-C₃-alkoxy-C₁-C₃-alkyl-, hydroxycarbonyl, hydroxycarbonyl-C₁-C₃-alkoxy-, C₁-C₃-alkoxycarbonyl-, C₁-C₃-alkoxycarbonyl-C₁-C₃-alkoxy-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkoxycarbonylamino-, C₁-C₃-alkylcarbonyl-C₁-C₃-alkylamino-, C₁-C₃-alkoxycarbonyl-C₁-C₃-alkylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, cyano and nitro, wherein said heterocyclyls are five or six membered heterocyclyls containing from one to three heteroatoms each independently selected from the group consisting of oxygen, nitrogen and sulphur, and wherein the aryl or heterocyclyl components are unsubstituted or substituted by one to four substituents each independently selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, phenyl, cyano and nitro;
R⁷ and R⁸ are each independently selected from the group consisting of hydrogen, halogen, C₁-C₃-alkyl-, C₁-C₃-alkoxy-, C₁-C₃-haloalkyl- and C₁-C₃-haloalkoxy-;
R⁹ is selected from the group consisting of hydrogen, hydroxyl, halogen, C₁-C₆-alkyl, C₃-C₆-cycloalkyl, C₁-C₆-haloalkyl, C₂-C₆-haloalkenyl, C₁-C₆-alkoxy-, C₂-C₆-alkenyloxy-, C₃-C₆-cycloalkyl-C₁-C₃-alkyl-, C₁-C₆- alkoxy-C₁-C₃-alkyl-, C₁-C₆-alkoxy-C₂-C₆-alkoxy-, C₁-C₆-alkoxy-C₂-C₆-alkoxy-C₁-C₃-alkyl-, C₁-C₆-haloalkoxy-, C₁-C₆-haloalkoxy-C₁-C₃-alkyl-, C₁-C₆-alkyl-S(O)ₙ-, C₁-C₆-haloalkyl-S(O)ₙ-, aryl, aryl-S(O)ₙ-, heterocyclyl, heterocyclyl-S(O)ₙ-, aryloxy-, aryl-C₂-C₆-alkyl-, aryl-C₁-C₆-alkoxy-, heterocyclyloxy-, heterocyclyl-C₁-C₃-alkoxy-C₁-C₃-alkyl-, hydroxycar-bonyl, hydroxycarbonyl-C₁-C₃-alkoxy-, C₁-C₃-alkoxycarbonyl-, C₁-C₃-alkoxycarbonyl-C₁-C₃-alkoxy-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkoxycarbonylamino-, C₁-C₃-alkylcarbonyl-C₁-C₃-alkylamino-, C₁-C₃-alkoxycarbonyl-C₁-C₃-alkylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, cyano and nitro, wherein said heterocyclyls are five or six membered heterocyclyls containing from one to three heteroatoms each independently selected from the group consisting of oxygen, nitrogen and sulphur, and wherein the aryl or heterocyclyl components are unsubstituted or substituted by one to four substituents each independently selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, phenyl, cyano and nitro;
   and wherein R⁵ and R⁹ can together form a saturated or unsaturated 5- or 6-membered carbocyclic or heterocyclic ring, said heterocyclic ring comprising one to three heteroatoms each in-dependently selected from nitrogen, oxygen and sulphur, the 5- or 6-membered ring being unsubstituted or substituted by one to four R¹¹; or
   R⁶ and R⁹ can together form a saturated or unsaturated 5- or 6-membered carbocyclic or heterocyclic ring, said heterocyclic ring comprising one to three heteroatoms each independently selected from nitrogen, oxygen and sulphur, the 5- or 6-membered ring being unsubstituted or substituted by one to four R¹¹; or
R⁶ and R⁸ can together form a saturated or unsaturated 5- or 6-membered carbocyclic or heterocyclic ring, said heterocyclic ring comprising one to three heteroatoms each independently selected from nitrogen, oxygen and sulphur, the 5- or 6-membered ring being unsubstituted or substituted by one to four R¹²;
R¹⁰ is selected from the group consisting of halo-, C₁-C₃-alkyl, C₁-C₃-haloalkyl and C₁-C₆-alkoxy;
R¹¹ is selected from the group of hydrogen, cyano, halo-, oxy-, C₁-C₃-alkyl-S(O)ₙ-, C₁-C₃-alkyl, C₂-C₃-alkenyl, C₂-C₃-alkynyl, C₁-C₃-alkoxy and C₁-C₃-haloalkyl;
R¹² is selected from the group of hydrogen, cyano, halo-, C₁-C₃-alkyl-S(O)ₙ-, C₁-C₃-alkyl, C₂-C₃-alkenyl, C₂-C₃-alkynyl, morpholinyl- and C₁-C₃-haloalkyl; and
n is 0, 1 or 2 .

The present invention also provides agrochemical compositions comprising at least one pyrazolone compound of formula (I) and auxiliaries customary for formulating crop protection agents.

The present invention also provides the use of the pyrazolone compound of formula (I) as herbicides, i.e. for controlling harmful plants.

The present invention furthermore provides a method for controlling unwanted vegetation where a herbicidally effective amount of at least one pyrazolone compound of the formula (I) is allowed to act on plants, their seeds and/or their habitat. Application can be done before, during and/or after, preferably during and/or after, the emergence of the undesirable plants.

Moreover, the invention relates to processes for preparing the pyrazolone compound of formula (I).

As used herein, the terms "controlling" and "combating" are synonyms. As used herein, the terms "undesirable vegetation" and "harmful plants" are synonyms.

If the compounds of formula (I) as described herein are capable of forming geometrical isomers, for example E/Z isomers, it is possible to use both, the pure isomers and mixtures thereof, in the compositions according to the invention.

If the compounds of formula (I) as described herein have one or more centres of chirality and, as a consequence, are present as enantiomers or diastereomers, it is possible to use both, the pure enantiomers and diastereomers and their mixtures, in the compositions according to the invention.

If the compounds of formula (I) as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methylammonium, isopropylammonium, dimethylammonium, diisopropylammonium, trime-thylammonium, heptylammonium, dodecylammonium, tetradecylammonium, tetramethylammo-nium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetri-amine.

Anions of useful acid addition salts are primarily chloride, bromide, fluoride, iodide, hydrogensul-fate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

Compounds of formula (I) as described herein having a carboxyl group can be employed in the form of the acid, in the form of an agriculturally suitable salt as mentioned above or else in the form of an agriculturally acceptable derivative, for example as amides, such as mono- and di-C₁-C₆-alkylamides or arylamides, as esters, for example as allyl esters, propargyl esters, C₁-C₁₀-alkyl esters, alkoxyalkyl esters, tefuryl ((tetrahydrofuran-2-yl)methyl) esters and also as thioesters, for example as C₁-C₁₀-alkylthio esters. Preferred mono- and di-C₁-C₆-alkylamides are the methyl and the dimethylamides. Preferred arylamides are, for example, the anilides and the 2-chloroanilides. Preferred alkyl esters are, for example, the methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄-alkoxy-C₁-C₄-alkyl esters are the straight-chain or branched C₁-C₄-alkoxy ethyl esters, for example the 2-methoxyethyl, 2-ethoxyethyl, 2-butoxyethyl (buto-tyl), 2-butoxypropyl or 3-butoxypropyl ester. An example of a straight-chain or branched C₁-C₁₀-alkylthio ester is the ethylthio ester.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention. The organic moieties mentioned in the definition of the variables R¹, R², R³, R⁴, R⁵, R⁶, R⁷, R⁸, R⁹, R¹⁰, R¹¹ and R¹², are - like the term halogen - collective terms for individual enumerations of the individual group members. The term halogen denotes in each case fluorine, chlorine, bromine or iodine. All hydrocarbon chains, e.g. alkyl, alkenyl or alkynyl chains, can be straight-chain or branched, the prefix Cₙ-Cₘ denoting in each case the possible number of carbon atoms in the group.

Examples of such meanings are:
- C₁-C₃-alkyl and also the C₁-C₃-alkyl moieties of C₁-C₆-alkoxy-C₁-C₃-alkyl, C₃-C₆-cycloalkyl-C₁-C₃-alkyl-, C₁-C₆-alkoxy-C₂-C₆-alkoxy-C₁-C₃-alkyl-, C₁-C₆-haloalkoxy-C₁-C₃-alkyl-, heterocyclyl-C₁-C₃-alkoxy-C₁-C₃-alkyl-, C₁-C₃-alkylamino-, C₁-C₃-dialkylamino-, C₁-C₃-alkylamino-S(O)n-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, C₁-C₃-alkylcarbonyl-C₁-C₃-alkylamino-, C₁-C₃-alkoxycarbonyl-C₁-C₃-alkylamino- and C₁-C₃-alkyl-S(O)ₙ-: for example CH₃, C₂H₅, n-propyl, and CH(CH₃)₂;
- C₁-C₄-alkyl: C₁-C₃-alkyl as mentioned above, and also, for example, n-butyl, CH(CH₃)-C₂H₅, CH₂-CH(CH₃)₂ and C(CH₃)₃;
- C₁-C₆-alkyl and also the C₁-C₆-alkyl moieties of C₁-C₆-alkylcarbonyl-, C₁-C₆-alkylcarbonyl-C₁-C₃-alkoxy-, C₁-C₆-alkyl-S(O)ₙ- and C₁-C₆-alkyl-S(O)ₙcarbonyl-: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- C₂-C₆-alkyl and also the C₂-C₆-alkyl moieties of aryl-C₂-C₆-alkyl-: C₁-C₆-alkyl as mentioned above except for CH₃;
- C₁-C₃-haloalkyl: a C₁-C₃-alkyl radical as mentioned above which is partially or fully substituted by fluorine, chlorine, bromine and/or iodine, i.e., for example, chloromethyl, dichloromethyl, trichloromethyl, fluoromethyl, difluoromethyl, trifluoromethyl, chlorofluorome-thyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pen-tafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl or 1-(bromomethyl)-2-bromoethyl;
- C₁-C₆-haloalkyl and also the C₁-C₆-haloalkyl moieties of C₁-C₆-haloalkyl-S(O)ₙ-: C₁-C₃-haloalkyl as mentioned above, and also, for example, 4-fluorobutyl, 4-chlorobutyl, 4-bromobutyl, nonafluorobutyl, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl or dode-cafluorohexyl;
- C₃-C₆-cycloalkyl and also the C₃-C₆-cycloalkyl moieties of C₃-C₆-cycloalkyl-C₁-C₃-alkyl: monocyclic saturated hydrocarbons having 3 to 6 ring members, such as cyclopropyl, cyclobutyl, cyclopentyl and cyclohexyl;
- C₂-C₃-alkenyl: for example ethenyl, 1-propenyl, 2-propenyl and 1-methylethenyl;
- C₂-C₆-alkenyl and also the C₂-C₆-alkenyl moieties of C₂-C₆-alkenyloxy: C₂-C₃-alkenyl as mentioned above, and also, for example, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl or 1-ethyl-2-methyl-2-propenyl;
- C₂-C₆-haloalkenyl: a C₂-C₆-alkenyl radical as mentioned above which is partially or fully substituted by fluorine, chlorine, bromine and/or iodine, for example 2-chloroprop-2-en-1-yl, 3-chloroprop-2-en-1-yl, 2,3-dichloroprop-2-en-1-yl, 3,3-dichloroprop-2-en-1-yl, 2,3,3-trichloro-2-en-1-yl, 2,3-dichlorobut-2-en-1-yl, 2-bromoprop-2-en-1-yl, 3-bromoprop-2-en-1-yl, 2,3-dibromoprop-2-en-1-yl, 3,3-dibromoprop-2-en-1-yl, 2,3,3-tribromo-2-en-1-yl or 2,3-dibromobut-2-en-1-yl;
- C₂-C₃-alkynyl: for example ethynyl, 1-propynyl and 2-propynyl;
- C₂-C₆-alkynyl: C₂-C₃-alkynyl as mentioned above, and also, for example, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl or 1-ethyl-1-methyl-2-propynyl;
- C₁-C₃-alkoxy and also the C₁-C₃-alkoxy moieties of C₁-C₆-alkoxycarbonyl-C₁-C₃-alkoxy-, C₁-C₆-alkylcarbonyl-C₁-C₃-alkoxy-, heterocyclyl-C₁-C₃-alkoxy-C₁-C₃-alkyl-, C₁-C₃-alkoxycarbonyl-, heterocyclyl-C₁-C₃-alkoxy-C₁-C₃-alkyl-, hydroxycarbonyl-C₁-C₃-alkoxy-, C₁-C₃-alkoxycarbonylamino- and C₁-C₃-alkoxycarbonyl-C₁-C₃-alkylamino-: methoxy, ethoxy, propoxy and 1-methylethoxy;
- C₁-C₆-alkoxy and also the C₁-C₆-alkoxy moieties of C₁-C₆-alkoxy-C₁-C₃-alkyl, C₁-C₆-alkoxycarbonyl-, C₁-C₆-alkoxycarbonyl-C₁-C₃-alkoxy-, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₃-alkyl- and aryl-C₁-C₆-alkoxy-C₁-C₃-alkoxy as mentioned above, and also, for example, butoxy, 1-methylpropoxy, 2-methylpropoxy, 1,1-dimethylethoxy pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy;
- C₂-C₆-alkoxy and also the C₂-C₆-alkoxy moieties of C₁-C₆-alkoxy-C₂-C₆-alkoxy- and C₁-C₆-alkoxy-C₂-C₆-alkoxy-C₁-C₃-alkyl-: C₁-C₆-alkoxy as mentioned above except for methoxy;
- C₁-C₃-haloalkoxy: for example OCH₂F, OCHF₂, OCF₃, OCH₂Cl, OCHCl₂, OCCl₃, chloro-fluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, OC₂F₅, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy;
- C₁-C₆-haloalkoxy and also the C₁-C₆-haloalkoxy moieties of C₁-C₆-haloalkoxy-C₁-C₃-alkyl-: C₁-C₃-haloalkoxy as mentioned above, and also, for example, 4-fluorobutoxy, 4-chlorobutoxy, 4-bromobutoxy, nonafluorobutoxy, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "five or six membered heterocyclyl containing one to three heteroatoms each independently selected from the group consisting of oxygen, nitrogen and sulphur" means, for example: pyridazin-3-yl, pyridazin-4-yl, pyrimidin-2-yl, pyrimidin-4-yl, pyrimidin-5-yl, pyrazin-2-yl, piperidin-1-yl, morpholin-4-yl, thiomorpholin-4-yl, pyrollidin-1-yl, 2-furyl, 3-furyl, 2-thienyl, 3-thienyl, pyrazol-1-yl, pyrazol-3-yl, pyrazol-4-yl, isoxazol-3-yl, isoxazol-4-yl, isoxazol-5-yl, isothia-zol-3-yl, isothiazol-4-yl, isothiazol-5-yl, imidazol-1-yl, imidazol-2-yl, imidazol-4-yl, oxazol-2-yl, oxazol-4-yl, oxazol-5-yl, thiazol-2-yl, thiazol-4-yl and thiazol-5-yl, imidazol-5-yl, oxazol-2-yl, thiazol-2-yl, thiazol-4-yl, thiazol-5-yl, pyridin-2-yl, pyridin-3-yl, pyridin-4-yl, pyrimidin-2-yl, pyrimidin-4-yl, pyrimidin-5-yl, pyridazin-4-yl, pyrazin-2-yl, [1 H]-tetrazol-5-yl and [2H]-tetrazol-5-yl, isoxazolin-3-yl, oxetan-3-yl, 2-tetrahydrofuryl, 3-tetrahydrofuryl, 1,2,4 triazolyl or 1,3,4 triazolyl. Moreover, the nitrogen and/or the sulfur atom(s) of the heterocyclic ring may optionally be oxidized.

The term "saturated or unsaturated 5- or 6-membered carbocyclic or heterocyclic ring, said heterocyclic ring comprising one to three heteroatoms each independently selected from nitrogen, oxygen and sulphur" means, for example: phenyl, pyridazin-3-yl, pyridazin-4-yl, pyrimidin-2-yl, pyrimidin-4-yl, pyrimidin-5-yl, pyrazin-2-yl, piperidin-1-yl, morpholin-4-yl, thiomorpholin-4-yl, pyrollidin-1-yl, 2-furyl, 3-furyl, 2-thienyl, 3-thienyl, pyrazol-1-yl, pyrazol-3-yl, pyrazol-4-yl, isoxazol-3-yl, isoxazol-4-yl, isoxazol-5-yl, isothiazol-3-yl, isothiazol-4-yl, isothiazol-5-yl, imidazol-1-yl, imidazol-2-yl, imidazol-4-yl, oxazol-2-yl, oxazol-4-yl, oxazol-5-yl, thiazol-2-yl, thiazol-4-yl and thiazol-5-yl, imidazol-5-yl, oxazol-2-yl, thiazol-2-yl, thiazol-4-yl, thiazol-5-yl, pyridin-2-yl, pyridin-3-yl, pyridin-4-yl, pyrimidin-2-yl, pyrimidin-4-yl, pyrimidin-5-yl, pyridazin-4-yl, pyrazin-2-yl, [1 H]-tetrazol-5-yl and [2H]-tetrazol-5-yl, isoxazolin-3-yl, oxetan-3-yl, 2-tetrahydrofuryl, 3-tetrahydrofuryl, 1,2,4 triazolyl or 1,3,4 triazolyl. Moreover, the nitrogen and/or the sulfur atom(s) of the heterocyclic ring may optionally be oxidized.

The term "aryl" refers to an unsaturated aromatic carbocyclic group of from 6 to 20 carbon atoms having a single ring (e.g., phenyl) or multiple condensed (fused) rings, wherein at least one ring is aromatic (e.g. naphthalenyl or dihydrophenanthrenyl). Examples of aryls include phenyl, naphthalenyl, anthracenyl, indenyl or phenanthrenyl. A preferred aryl group is phenyl.

The preferred embodiments of the invention mentioned herein below have to be understood as being preferred either independently from each other or in combination with one another.

According to a preferred embodiment of the invention preference is also given to those compounds of formula I, wherein the variables, either independently of one another or in combination with one another, have the following meanings:

Preferably, R¹ and R² are each independently selected from the group consisting of C₁-C₆-alkyl, C₁-C₆-haloalkyl, C₃-C₆-cycloalkyl, C₁-C₆-alkoxy-C₁-C₃-alkyl-, C₃-C₆-cycloalkyl-C₁-C₃-alkyl- and oxetanyl.

More preferably, R¹ and R² are each independently selected from the group consisting of C₁-C₄-alkyl, cyclopropyl, oxetanyl, cyclopropylmethyl, difluoromethyl and trifluoroethyl.

Even more preferably, R¹ and R² are each independently selected from the group consisting of C₁-C₄-alkyl, cyclopropyl, oxetanyl, cyclopropylmethyl, difluoromethyl and trifluoroethyl.

In another preferred embodiment, R³ is selected from the group consisting of A1a, A1 b, A1 c, A1d, A1e, A1f, A1g, A1h, A1i, A1j, A2a, A2b and A2c as shown below 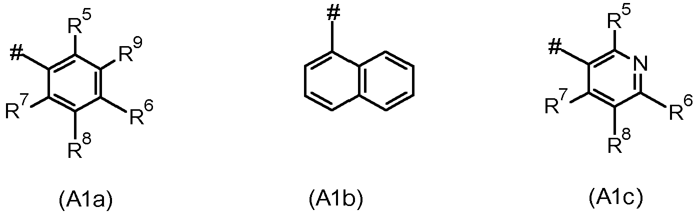 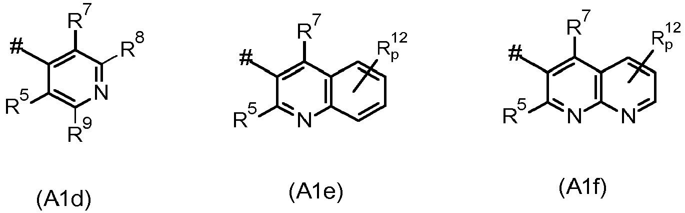 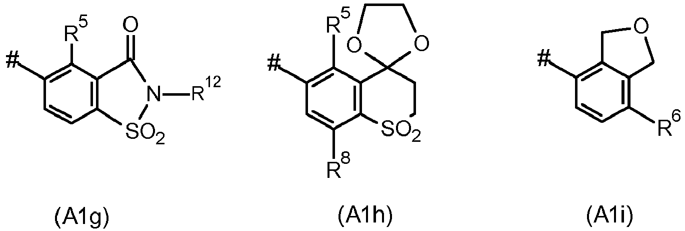 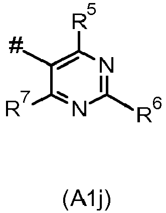 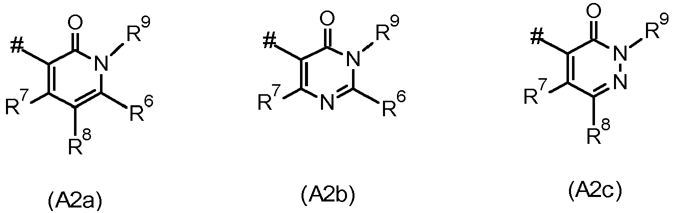 wherein #, R⁵, R⁶, R⁷, R⁸, R⁹ and R¹² are as previously defined and p is 0, 1, 2 or 3.

More preferably, R³ is selected from the group consisting of A1a, A1 c and A1d wherein #, R⁵, R⁶, R⁷, R⁸ and R⁹ are as previously defined.

Even more preferably, R³ is selected from the group consisting of phenyl, 3-pyridyl and 4-pyridyl.

Preferably, R⁵ is selected from the group consisting of hydroxyl, halogen, C₁-C₆-alkyl, C₃-C₆-cycloalkyl, C₁-C₆-haloalkyl, C₁-C₆-alkoxy, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₃-alkyl-, C₁-C₆-haloalkoxy, C₁-C₆-haloalkoxy-C₁-C₃-alkyl-, C₁-C₆-alkyl-S(O)n-, C₁-C₆-haloalkyl-S(O)ₙ-, heterocyclyl, heterocyclyl-C₁-C₃-alkoxy-C₁-C₃-alkyl-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkylcarbonyl-C₁-C₃-alkylamino-, C₁-C₃-alkyl-S(O)ₙ-amino-, C₁-C₃-alkyl-S(O)ₙ-C₁-C₃-alkylamino-, cyano and nitro, wherein said heterocyclyls are five or six membered heterocyclyls containing from one to three heteroatoms each independently selected from the group consisting of oxygen, nitrogen and sulphur, and wherein the heterocyclyl components may be unsubstituted or substituted by one to three substituents each independently selected from the group consisting of halo, C₁-C₃-alkyl, C₁-C₃-haloalkyl, C₁-C₃-alkoxy, C₁-C₃-haloalkoxy, cyano and nitro.

More preferably, R⁵ is selected from the group consisting of chloro, fluoro, methyl, trifluoromethyl, 2-fluoroethyl-, methoxyethoxymethyl-, trifluoromethoxymethyl-, methyl-S(O)ₙ-, ethyl-S(O)ₙ-, cyclopropyl, aryl, isoxazolinyl, morpholinyl, methyl-S(O)ₙ-dimethylamino-, cyano and nitro, wherein the heterocyclyl components may be unsubstituted or substituted by one to three substituents each independently selected from methyl and trifluoromethyl.

Even more preferably, R⁵ is selected from the group consisting of chloro, methyl, methyl-S(O)₂-, trifluoromethyl and cyclopropyl.

Preferably, R⁶ is selected from the group consisting of hydrogen, halogen, C₁-C₆-alkyl, C₁-C₆-haloalkyl, C₁-C₆-alkyl-S(O)ₙ-, C₃-C₆-cycloalkyl, C₁-C₆-alkoxy-, C₁-C₆-haloalkoxy, C₂-C₆-alkenyloxy-, C₃-C₆-cycloalkyl-C₁-C₃-alkyl-, C₁-C₆-alkoxy-C₁-C₃-alkyl-, C₁-C₆-alkoxy-C₂-C₆-alkoxy-, nitro and phenyl wherein the phenyl may be optionally substituted by one to four substituents selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, phenyl, cyano and nitro.

More preferably, R⁶ is selected from the group consisting of hydrogen, halogen, C₁-C₆-alkyl, C₁-C₆-haloalkyl, C₁-C₆-alkyl-S(O)ₙ- and C₃-C₄-cycloalkyl.

Even more preferably, R⁶ is selected from the group consisting of methyl, ethyl, chloro, trifluoromethyl, methyl-S(O)ₙ- and cyclopropyl.

Preferably, R⁷ is selected from the group consisting of hydrogen, halogen and C₁-C₃-alkyl. Most preferably, R⁷ is selected from the group consisting of hydrogen, fluorine, chlorine and methyl.

Preferably, R⁸ is selected from the group consisting of hydrogen, halogen and C₁-C₃-alkyl. Most preferably R⁸ is hydrogen.

Preferably, R⁹ is selected from the group consisting of hydrogen, halogen, C₁-C₆-alkyl, C₁-C₆-haloalkyl, C₁-C₆-alkyl-S(O)ₙ-, C₃-C₆-cycloalkyl, C₁-C₆-alkoxy-, C₁-C₆-haloalkoxy, C₂-C₆-alkenyloxy-, C₃-C₆-cycloalkyl-C₁-C₃-alkyl-, C₁-C₆-alkoxy-C₁-C₃-alkyl-, C₁-C₆-alkoxy-C₂-C₆-alkoxy-, nitro, 4,5-dihydroisoxazol-3-yl and phenyl wherein the phenyl may be optionally substituted by one to four substituents selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, phenyl, cyano and nitro.

More preferably, R⁹ is selected from the group consisting of hydrogen, 4,5-dihydroisoxazol-3-yl, halogen, C₁-C₆-alkyl, C₁-C₆-haloalkyl and C₁-C₆-alkyl-S(O)ₙ-.

Even more preferably, R⁹ is hydrogen or 4,5- dihydroisoxazol-3-yl.

A further embodiment relates to the N-oxides of the compounds of the formula I.
A further embodiment relates to salts of the compounds of the formula I, in particular those which are obtainable by quaternization of a nitrogen atom, which may preferably take place by alkylation of the compounds of the formula I. Preferred salts of the compounds are thus the N-alkyl salts, in particular the N-methyl salts.

The pyrazolone compounds of formula I according to the invention can be prepared by standard processes of organic chemistry, for example by the following processes:

The key intermediate of the formula (VI) can be prepared starting from the β-ketoesters of formula II (see Scheme 1 below) or the acetylenic esters of the formula VII (see Scheme 2 below) and will then be transformed to the desired pyrazolone compound I following the Scheme 3 below.

As shown in Scheme 1 below, the intermediate of the formula (VI) can be prepared via a three-step synthesis using the β-ketoester of formula II (wherein R is methyl or ethyl) as a starting material. In the first step, the intermediate III is synthesized by a ring closure reaction of the β-ketoester of formula II (wherein R is methyl or ethyl) and a hydrazine of the formula R¹NHNH₂. The starting β-ketoesters II and the hydrazines R¹NHNH₂ are commercially available and can be prepared according to methods well-known in the art. The reaction of the β-ketoester II with the hydrazine R¹NHNH₂ is typically conducted in an appropriate solvent such as e.g. methanol, ethanol, acetonitrile, toluene, pyridine or the like at a temperature between 20°C and 200°C, optionally in the presence of an acid such as p-toluenesulfonic acid, acetic acid or formic acid in an analogous manner to the procedures described in WO 2011/051958 and WO 2011/046774. The β-ketoester II and the hydrazine R¹NHNH₂ are generally reacted with one another in equimolar amounts. In the second step, the intermediates III are then reacted with a halogenating agent to give the intermediate IV. Suitable halogenating agents include, for example, N-halosuccinimides (e.g. N-chlorosuccinimide, N-bromosuccinimide or N-iodosuccinimide). The reaction of intermediate III with the halogenating agent is usually carried out in an organic solvent, such as, for example, N,N-dimethylformamide, acetonitrile, dichloromethane, chloroform, tetrahydrofurane, dioxane or the like at a temperature of from 0°C to 100°C. Optionally an organic base such as pyridine, triethylamine, N,N-dimethylaniline, or the like can be added. The halogenating agent is generally employed in an equimolar amount or in slight excess relative to the intermediate III.
In the third step, the intermediate VI is then obtained by reacting the intermediate IV with the compound R²Lg wherein Lg means an appropriate leaving group, such as, for example, chloro, bromo, iodo or sulfonate esters (e.g. tosylate and mesylate). The reaction of the intermediate IV with the compound R²Lg is usually conducted in an organic solvent, such as, for example, N,N-dimethylformamide, dimethylsulfoxide or acetone at a temperature of from 20°C to 100°C in the presence of a base such as e.g. potassium carbonate, sodium carbonate, sodium hydroxide, potassium hydroxide or tert-butoxide. The compound R²Lg is generally employed in an equimolar amount or in slight excess relative to the intermediate IV.
Alternatively, the substituent R² can first be introduced into the pyrazolone ring by reacting the intermediate III with the compound R²Lg to give an intermediate of formula V. The intermediate V is then converted to the intermediate VI by reaction with a halogenating agent. Essentially the same leaving groups, halogenating agents and reaction conditions as those stated above can be used in this alternative reaction sequence.

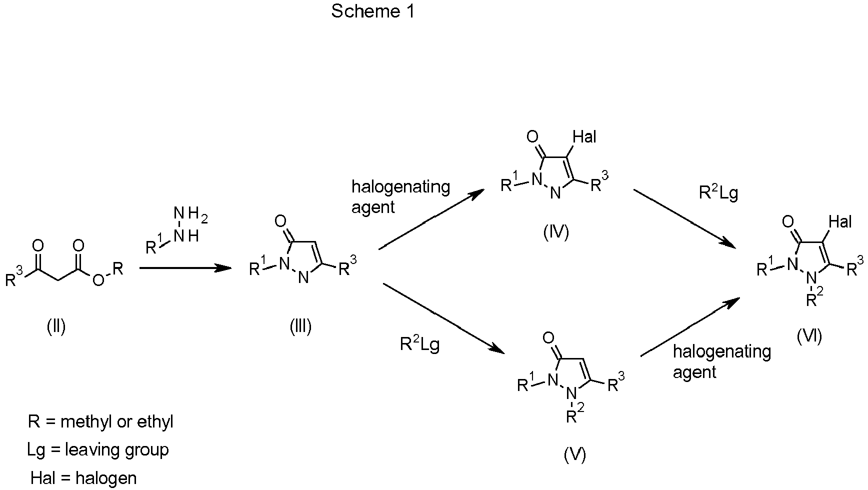

As shown in Scheme 2 below, the intermediate of the formula (VI) can also be prepared via a three-step synthesis using the acetylenic esters VII (wherein R is methyl or ethyl) as a starting material. In the first step, the acetylenic esters VII are reacted with a hydrazine of the formula R²NHNH2 to give the intermediates VIII. The starting acetylenic esters VII and the hydrazines R²NHNH₂ are commercially available and can be prepared according to methods well-known in the art. The reaction of the acetylenic ester VII with the hydrazine R²NHNH₂ is typically conducted in an appropriate solvent such as e.g. ethanol, tert-butanol, a mixture of water and methanol (e.g. 1:1 (v/v)), and dichloromethane at a temperature between 0°C and 20°C, optionally in the presence of a base such as potassium hydroxide or potassium tert-butoxide, in an analogous manner to the procedures disclosed in EP0680954, US 2007/049574 and Journal of Organic Chemistry 1992, 57, 5680-5686. The acetylenic esters VII and the hydrazine R²NHNH₂ are generally reacted with one another in equimolar amounts. In the second step, the intermediates VIII are then reacted with a halogenating agent to give the intermediate IX. Suitable halogenating agents include, for example, N-halosuccinimides (e.g. N-chlorosuccinimide, N-bromosuccinimide or N-iodosuccinimide). The reaction of intermediate VIII with the halogenating agent is usually carried out in an organic solvent, such as, for example, N,N-dimethylformamide, acetonitrile, dichloromethane, chloroform, tetrahydrofurane, dioxane or the like at a temperature of from 0°C to 100°C. Optionally an organic base such as pyridine, triethylamine, N,N-dimethylaniline, or the like can be added. The halogenating agent is generally employed in an equimolar amount or in slight excess relative to the intermediate VIII.

In the third step, the intermediate VI is then obtained by reacting the intermediate IX with the compound R¹Lg wherein Lg means an appropriate leaving group, such as, for example, chloro, bromo, iodo or sulfonate esters (e.g. tosylate and mesylate). The reaction of the intermediate IX with the compound R¹Lg is usually conducted at a temperature of from 20°C to 100°C in an organic solvent in the presence of a base such as, for example, potassium carbonate, sodium carbonate, sodium hydroxide, potassium hydroxide or tert-butoxide. Suitable organic solvents include N,N-dimethylformamide, dimethylsulfoxide or acetone. The compound R¹Lg is generally employed in an equimolar amount or in slight excess relative to the intermediate IV. The bases are generally employed in equimolar amounts; however, they can also be used in excess or, if appropriate, as solvents.

Alternatively, the substituent R¹ can first be introduced into the pyrazolone ring by reacting the intermediate VIII with the compound R¹Lg to give the intermediate of formula V. The intermediate V is then converted to the intermediate VI by reaction with a halogenating agent. Essentially the same leaving groups, halogenating agents and reaction conditions as those stated above can be used in this alternative reaction sequence.

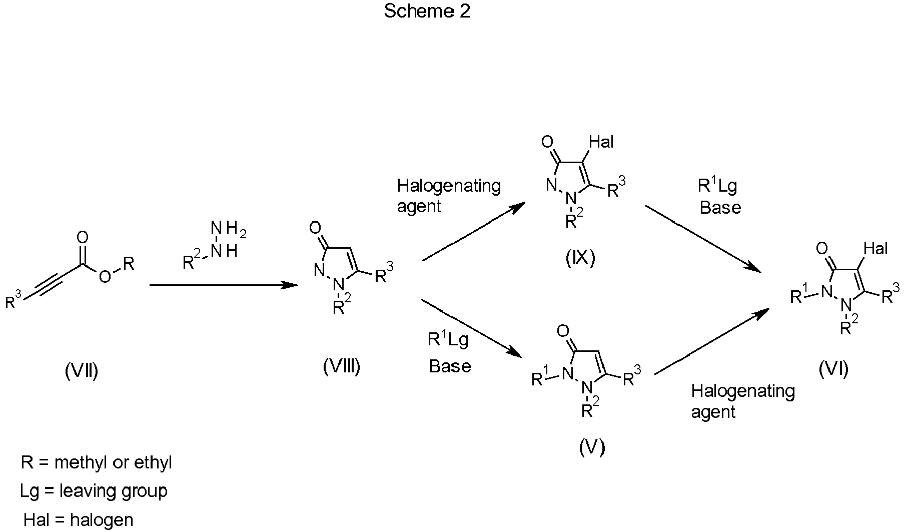

As shown in Scheme 3 below, the intermediate VI is subsequently transformed to the hydroxy compounds I.1 (which correspond to compounds of the formula I with R⁴ = H) in the presence of potassium hydroxide (KOH) and a phase transfer catalyst such as, for example, benzyltrimethylammonium hydroxide. The hydroxylation reaction is usually carried out at a temperature of from 20°C to 120 °C in an organic solvent. Suitable organic solvents are toluene and benzene. The amount of KOH is generally at least 20 molar equivalents relative to the intermediate VI. The amount of the phase transfer catalyst is generally at least 5 molar equivalents relative to the intermediate VI. The hydroxy compounds I.1 can then be converted to the compounds I by reaction with R⁴Lg wherein R⁴ is different from hydrogen and Lg means a leaving group. Suitable leaving groups include chloro, bromo, iodo and sulfonate esters (e.g. tosylate and mesylate). The reaction of the hydroxy compound I.1 with the compound R⁴Lg is usually conducted at a temperature of from 0°C to 100°C in an organic solvent in the presence of a base such as, for example, potassium carbonate, sodium carbonate, sodium hydroxide, potassium hydroxide, and potassium tert-butoxide. Suitable organic solvents include N,N-dimethylformamide, dimethyl sulfoxide, acetone or acetonitrile. The compound R⁴Lg is generally employed in an equimolar amount or in slight excess relative to the hydroxy compound I.1. The bases are generally employed in equimolar amounts; however, they can also be used in excess or, if appropriate, as solvents.

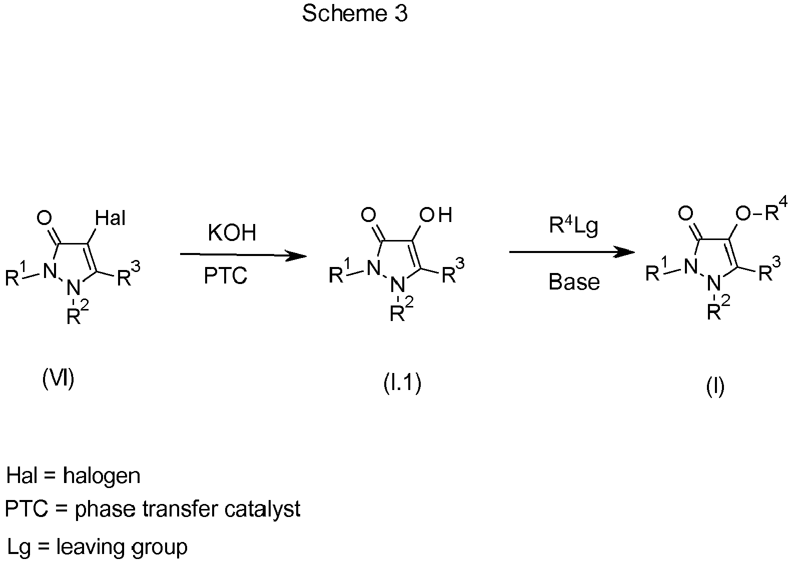

With respect to the variables, preferred embodiments of the β-ketoester II, the acetylenic ester VII, the hydrazine R¹NHNH₂, the hydrazine R²NHNH₂, the compound R¹-Lg, the compound R²-Lg, the compound R⁴-Lg and the intermediates III, IV, V, VI, VII, VIII and IX correspond to those described above for the variables of the compound of formula I.

The reaction mixtures are worked up in a customary manner, for example by mixing with water, separating the phases and, if appropriate, chromatographic purification of the crude products. Some of the intermediates and end products are obtained in the form of colorless or slightly brownish viscous oils which are purified or freed from volatile components under reduced pressure and at moderately elevated temperature. If the intermediates and end products are obtained as solids, the purification can also be carried out by recrystallization or digestion.
If individual compounds I cannot be obtained by the routes described above, they can be prepared by derivatization of other compounds I.
If the synthesis yields mixtures of isomers, a separation is generally however not necessarily required since in some cases the individual isomers can be interconverted during work-up for use or during application (for example under the action of light, acids or bases). Such conversions may also take place after application, for example in the case of the treatment of plants in the treated plant or in the harmful plant to be controlled.

To widen the spectrum of action and to achieve synergistic effects, the compounds of formula (I) may be mixed with a large number of representatives of other herbicidal or growth-regulating active ingredient groups and then applied concomitantly. Suitable components for mixtures are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils and ureas.

It may furthermore be beneficial to apply the compounds of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

Moreover, it may be useful to apply the compounds of formula (I) in combination with safeners. Safeners are chemical compounds which prevent or reduce damage on useful plants without having a major impact on the herbicidal action of the compounds of the formula (I) towards unwanted plants. They can be applied either before sowings (e.g. on seed treatments, shoots or seedlings) or in the pre-emergence application or post-emergence application of the useful plant. The safeners and the compounds of formula (I) and optionally the herbicides B can be applied simultaneously or in succession.

Suitable safeners are e.g. (quinolin-8-oxy)acetic acids, 1-phenyl-5-haloalkyl-1 H-1,2,4-triazol-3-carboxylic acids, 1-phenyl-4,5-dihydro-5-alkyl-1 H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

The invention also relates to agrochemical compositions comprising at least an auxiliary and at least one compound of formula (I) according to the invention.

An agrochemical composition comprises a pesticidally effective amount of a compound of formula (I). The term "effective amount" denotes an amount of the composition or of the compounds I, which is sufficient for controlling unwanted plants, especially for controlling unwanted plants in cultivated plants and which does not result in a substantial damage to the treated plants. Such an amount can vary in a broad range and is dependent on various factors, such as the plants to be controlled, the treated cultivated plant or material, the climatic conditions and the specific compound of formula (I) used.

The compound of formula (I), their N-oxides, salts or derivatives can be converted into customary types of agrochemical compositions, e. g. solutions, emulsions, suspensions, dusts, powders, pastes, granules, pressings, capsules, and mixtures thereof. Examples for agrochemical composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical compositions types are defined in the "Catalogue of pesticide formulation types and international coding system", Technical Monograph No. 2, 6th Ed. May 2008, CropLife International.

The agrochemical compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compounds of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for agrochemical composition types and their preparation are:
i) Water-soluble concentrates (SL, LS)
   10-60 wt% of a compound of formula (I) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound of formula (I) according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of compound of formula (I) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of compound of formula (I) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound of formula (I) according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound of formula (I) according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula (I) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound of formula (I) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a compound of formula (I) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a compound of formula (I) according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
ix) Dustable powders (DP, DS)
   1-10 wt% of a compound of formula (I) according to the invention are ground finely and mixed intimately with solid carrier (e.g. finely divided kaolin) ad 100 wt%.
x) Granules (GR, FG)
   0.5-30 wt% of a compound of formula (I) according to the invention is ground finely and associated with solid carrier (e.g. silicate) ad 100 wt%. Granulation is achieved by extrusion, spray-drying or the fluidized bed.
xi) Ultra-low volume liquids (UL)
   1-50 wt% of a compound of formula (I) according to the invention are dissolved in organic solvent (e.g. aromatic hydrocarbon) ad 100 wt%.

The agrochemical compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of the compound of formula (I). The compound of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The agrochemical compositions in question give, after two-to-tenfold dilution, active substance concentrations of from 0.01 to 60% by weight, preferably from 0.1 to 40% by weight, in the ready-to-use preparations. Application can be carried out before or during sowing.

Methods for applying compounds of formula (I) or agrochemical compositions thereof, on to plant propagation material, especially seeds, include dressing, coating, pelleting, dusting, soaking and in-furrow application methods of the propagation material. Preferably, compounds of formula (I) or agrochemical compositions thereof, respectively, are applied on to the plant propagation material by a method such that germination is not induced, e. g. by seed dressing, pelleting, coating and dusting.

Various types of oils, wetters, adjuvants, fertilizer, or micronutrients, and further pesticides (e.g. herbicides, insecticides, fungicides, growth regulators, safeners) may be added to the compounds of formula (I) or the agrochemical compositions comprising them as premix or, if appropriate not until immediately prior to use (tank mix). These agents can be admixed with the agrochemical compositions according to the invention in a weight ratio of 1:100 to 100:1, preferably 1:10 to 10:1.

The user applies the compounds of formula (I) according to the invention or the agrochemical compositions comprising them usually from a pre-dosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, either individual components of the agrochemical composition according to the invention or partially premixed components, e. g. components comprising compounds of formula (I), may be mixed by the user in a spray tank and further auxiliaries and additives may be added, if appropriate.

In a further embodiment, individual components of the agrochemical composition according to the invention such as parts of a kit or parts of a binary or ternary mixture may be mixed by the user himself in a spray tank and further auxiliaries may be added, if appropriate.

In a further embodiment, either individual components of the agrochemical composition according to the invention or partially premixed components, e. g components comprising compounds of formula (I) can be applied jointly (e.g. after tank mix) or consecutively.

The compounds of formula (I) are suitable as herbicides. They are suitable as such, as an appropriately formulated composition (agrochemical composition).

The compounds of formula (I) or the agrochemical compositions comprising them control vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

The compounds of formula (I) or the agrochemical compositions comprising them are applied to the plants mainly by spraying the leaves. Here, the application can be carried out using, for example, water as carrier by customary spraying techniques using spray liquor amounts of from about 100 to 1000 l/ha (for example from 300 to 400 l/ha). The compounds of formula (I) or the agrochemical compositions comprising them, may also be applied by the low-volume or the ultra-low-volume method, or in the form of microgranules.

Application of the compounds of formula (I) or the agrochemical compositions comprising them can be done before, during and/or after, preferably during and/or after, the emergence of the undesirable plants.

The compounds of formula (I) or the agrochemical compositions comprising them can be applied pre-, post-emergence or pre-plant, or together with the seed of a crop plant. It is also possible to apply the compounds of formula (I) or the agrochemical compositions comprising them, by applying seed, pretreated with the compounds of formula (I) or the agrochemical compositions comprising them, of a crop plant. If the active ingredients are less well tolerated by certain crop plants, application techniques may be used in which the agrochemical compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the compounds of formula (I) or the agrochemical compositions comprising them can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the compounds of formula (I) or the agrochemical compositions prepared therefrom. Here, the agrochemical compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

When employed in plant protection, the amounts of active substances applied, i.e. the compounds of formula (I), without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of the compounds of formula (I) is from 0.001 to 3 kg/ha, preferably from 0.005 to 2.5 kg/ha and in particular from 0.01 to 2 kg/ha of active substance (a.s.).

In another preferred embodiment of the invention, the rates of application of the compounds of formula (I) according to the present invention (total amount of compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the compounds of formula (I) is 0.1 to 1000 g/ha, preferably1 to 750 g/ha, more preferably 5 to 500 g/ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. the compounds of formula (I), are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Depending on the application method in question, the compounds of formula (I) or the agrochemical compositions comprising them can additionally be employed in a further number of crop plants for eliminating undesirable plants. Examples of suitable crops are the following: Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays.

Especially preferred crops are crops of cereals, corn, soybeans, rice, oilseed rape, cotton, potatoes, peanuts or permanent crops.

The compounds of formula (I) according to the invention or the agrochemical compositions comprising them, can also be used in genetically modified plants. The term "genetically modified plants" is to be understood as plants whose genetic material has been modified by the use of recombinant DNA techniques to include an inserted sequence of DNA that is not native to that plant species' genome or to exhibit a deletion of DNA that was native to that species' genome, wherein the modification(s) cannot readily be obtained by cross breeding, mutagenesis or natural recombination alone. Often, a particular genetically modified plant will be one that has obtained its genetic modification(s) by inheritance through a natural breeding or propagation process from an ancestral plant whose genome was the one directly treated by use of a recombinant DNA technique. Typically, one or more genes have been integrated into the genetic material of a genetically modified plant in order to improve certain properties of the plant. Such genetic modifications also include but are not limited to targeted post-translational modification of protein(s), oligo- or polypeptides. e. g., by inclusion therein of amino acid mutation(s) that permit, decrease, or promote glycosylation or polymer additions such as prenylation, acetylation farnesylation, or PEG moiety attachment.

Plants that have been modified by breeding, mutagenesis or genetic engineering, e.g. have been rendered tolerant to applications of specific classes of herbicides, such as auxin herbicides such as dicamba or 2,4-D; bleacher herbicides such as hydroxyphenylpyruvate dioxygenase (HPPD) inhibitors or phytoene desaturase (PDS) inhibitors; acetolactate synthase (ALS) inhibitors such as sulfonyl ureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by mutagenesis and conventional methods of breeding, e. g., Clearfield® summer rape (Canola, BASF SE, Germany) being tolerant to imidazolinones, e. g., imazamox, or ExpressSun® sunflowers (DuPont, USA) being tolerant to sulfonyl ureas, e. g., tribenuron. Genetic engineering methods have been used to render cultivated plants such as soybean, cotton, corn, beets and rape, tolerant to herbicides such as glyphosate, imidazolinones and glufosinate, some of which are under development or commercially available under the brands or trade names RoundupReady® (glyphosate tolerant, Monsanto, USA), Cultivance® (imidazolinone tolerant, BASF SE, Germany) and LibertyLink® (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as delta-endotoxins, e. g., CryIA(b), CryIA(c), CryIF, CryIF(a2), CryIIA(b), CryIIIA, CryIIIB(b1) or Cry9c; vegetative insecticidal proteins (VIP), e. g., VIP1, VIP2, VIP3 or VIP3A; insecticidal proteins of bacteria colonizing nematodes, e. g., Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as including pre-toxins, hybrid proteins, truncated or otherwise modified proteins. Hybrid proteins are characterized by a new combination of protein domains, (see, e. g., WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g., in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, Knock-Out®, BiteGard®, Protecta®, Bt11 (e. g., Agrisure® CB) and Bt176 from Syngenta Seeds SAS, France, (corn cultivars producing the Cry1Ab toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e.g., EP-A 392 225), plant disease resistance genes (e. g., potato culti-vars, which express resistance genes acting against Phytophthora infestans derived from the Mexican wild potato, Solanum bulbocastanum) or T4-lyso-zym (e.g., potato cultivars capable of synthesizing these proteins with increased resistance against bacteria such as Erwinia amylovora). The methods for producing such genetically modi-fied plants are generally known to the person skilled in the art and are described, e.g., in the publications mentioned above.

Furthermore, plants are also covered that are by the use of recombinant DNA techniques capable to synthesize one or more proteins to increase the productivity (e.g., bio-mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of ingredients or new ingredients, specifically to improve human or animal nutrition, e. g., oil crops that produce health-promoting long-chain omega-3 fatty acids or unsaturated omega-9 fatty acids (e. g., Nexera® rape, Dow AgroSciences, Canada).

Furthermore, plants are also covered that contain by the use of recombinant DNA techniques a modified amount of ingredients or new ingredients, specifically to improve raw material production, e.g., potatoes that produce increased amounts of amylopectin (e.g. Amflora® potato, BASF SE, Germany).

Furthermore, it has been found that the compounds of formula (I) according to the invention or the agrochemical compositions comprising them are also suitable for the defoliation and/or desiccation of plant parts, for which crop plants such as cotton, potato, oilseed rape, sunflower, soybean or field beans, in particular cotton, are suitable. In this regard, agrochemical compositions for the desiccation and/or defoliation of plants, processes for preparing these agrochemical compositions and methods for desiccating and/or defoliating plants using the compounds of formula (I) have been found.

As desiccants, the compounds of formula (I) are particularly suitable for desiccating the above-ground parts of crop plants such as potato, oilseed rape, sunflower and soybean, but also cereals. This makes possible the fully mechanical harvesting of these important crop plants. Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

### Use examples

The herbicidal activity of the compounds of formula (I) is demonstrated by the following greenhouse experiments:

The culture containers used are plastic flowerpots containing loamy sand with approximately 3.0% of humus as the substrate. The seeds of the test plants are sown separately for each species.

For the pre-emergence treatment, the active ingredients, which have been suspended or emulsified in water, are applied directly after sowing by means of finely distributing nozzles. The containers are irrigated gently to promote germination and growth and subsequently covered with transparent plastic hoods until the plants have rooted. This cover causes uniform germination of the test plants, unless this has been impaired by the active ingredients.

For the post-emergence treatment, the test plants are first grown to a height of 3 to 15 cm, depending on the plant habit, and only then treated with the active ingredients which have been suspended or emulsified in water. For this purpose, the test plants are either sown directly and grown in the same containers, or they are first grown separately as seedlings and transplanted into the test containers a few days prior to treatment.

Depending on the species, the plants are kept at 10 - 25°C or 20 - 35°C, respectively.

The test period extends over 2 to 4 weeks. During this time, the plants are tended, and their response to the individual treatments is evaluated.

Evaluation is carried out using a scale from 0 to 100. 100 means no emergence of the plants, or complete destruction of at least the aerial moieties, and 0 means no damage, or normal course of growth. A good herbicidal activity is given at values of at least 70 and a very good herbicidal activity is given at values of at least 85.

The plants used in the greenhouse experiments can be selected from any of the following species:

| Bayer code | Scientific name |
|---|---|
| ABUTH | Abutilon theophrasti |
| ALOMY | Alopercurus myosuroides |
| AMARE | Amaranthus retroflexus |
| AMBEL | Ambrosia artemisiifolia |
| APESV | Apera spica-venti |
| BIDPI | Bidens pilosa |
| BRADE | Brachiaria deflexa |
| BRAPL | Brachiaria plantaginea |
| CHEAL | Chenopodium album |
| COMBE | Commenline benghalensis |
| DIGSA | Digitaria sanguinales |
| ECHCG | Echinocloa crus-galli |
| ELEIN | Eleusine indica |
| ERBVI | Eriochloa villosa |
| ERICA | Erigeron canadensis |
| LAMAM | Lamium amplexicaule |
| LAMPU | Lamium purpureum |
| PANDI | Panicum dichotomiflorun |
| PHBPU | Pharbitis purpurea |
| POAAN | Poa annua |
| POLCO | Polygonum convolvulus |
| SETLU | Setaria lutescens |
| SETFA | Setaria faberi |
| SETVE | Setaria verticillata |
| SETVI | Setaria viridis |
| SOLNI | Solanum nigrum |
| SORHA | Sorghum halepense |
| STEME | Stellaria media |
| VERPE | Veronica persica |

## Claims

1. A pyrazolone compound of the formula I 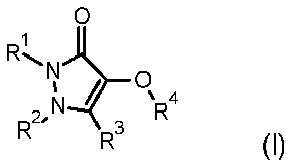 or an agriculturally suitable salt or N-oxide thereof,
wherein
R¹ and R² are each independently selected from the group consisting of hydrogen, C₁-C₆-alkyl, C₃-C₆-cycloalkyl, C₁-C₆-haloalkyl, C₁-C₆-alkoxy-C₁-C₃-alkyl, C₃-C₆-cydoalkyl-C₁-C₃-alkyl-, oxetanyl and tetrahydropyranyl;
R³ is a group represented by the formula A1 or A2 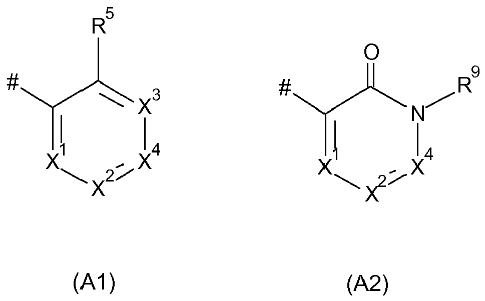
wherein in each of the formulae A1 and A2
# denotes the bonding site to the remainder of the formula (I);
X¹ is N or CR7;
X² is N or CR⁸;
X³ is N or CR⁹;
X⁴ is N or CR⁶;
R⁴ is selected from the group consisting of hydrogen, C₁-C₆-alkylcarbonyl-, arylcarbonyl-, C₁-C₆-alkoxycarbonyl-, C₁-C₆-alkoxycarbonyl-C₁-C₃-alkoxy-, C₁-C₆-alkylcarbonyl-C₁-C₃-alkoxy-, C₁-C₆-alkyl-S(O)ₙ-, C₁-C₆-alkyl-S(O)ₙcarbonyl- and aryl-S(O)ₙ-, wherein said aryl groups are unsubstituted or substituted by one to seven R¹⁰;
R⁵ is selected from the group consisting of hydroxyl, halogen, C₁-C₆-alkyl, C₃-C₆-cycloalkyl, C₁-C₆-haloalkyl, C₁-C₆-alkoxy-, C₃-C₆-cycloalkyl-C₁-C₃-alkyl-, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₃-alkyl-, C₁-C₆-haloalkoxy-, C₁-C₆-haloalkoxy-C₁-C₃-alkyl-, C₁-C₆-alkyl-S(O)ₙ-, C₁-C₆-haloalkyl-S(O)ₙ-, aryl-S(O)ₙ-, heterocyclyl, heterocyclyl-S(O)ₙ-, heterocyclyloxy-, heterocyclyl-C₁-C₃-alkoxy-C₁-C₃-alkyl-, C₁-C₃-alkoxycarbonyl-, C₁-C₃-alkoxycarbonyl-C₁-C₃-alkoxy-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, cyano and nitro, wherein said heterocyclyls are five or six membered heterocyclyls containing from one to three heteroatoms each independently selected from the group consisting of oxygen, nitrogen and sulphur, and wherein the aryl or heterocyclyl components may be unsubstituted or substituted by one to four substituents each independently selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, cyano and nitro;
R⁶ is selected from the group consisting of hydrogen, hydroxyl, halogen, C₁-C₆-alkyl, C₃-C₆-cycloalkyl, C₁-C₆-haloalkyl, C₂-C₆-alkenyl, C₂-C₆-haloalkenyl, C₂-C₆-alkynyl, C₁-C₆-alkoxy-, C₂-C₆-alkenyloxy-, C₃-C₆-cycloalkyl-C₁-C₃-alkyl-, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₆-alkyl-, C₁-C₆-haloalkoxy-, C₁-C₆-haloalkoxy-C₁-C₃-alkyl-, C₁-C₆-alkyl-S(O)ₙ-, C₁-C₆-haloalkyl-S(O)ₙ-, aryl, aryl-S(O)ₙ-, heterocyclyl, heterocyclyl-S(O)ₙ-, aryloxy-, aryl-C₂-C₆-alkyl-, aryl-C₁-C₆-alkoxy-, heterocyclyloxy-, heterocyclyl-C₁-C₃-alkoxy-C₁-C₃-alkyl-, hydroxycarbonyl, hydroxycarbonyl-C₁-C₃-alkoxy-, C₁-C₃-alkoxycarbonyl-, C₁-C₃-alkoxycarbonyl-C₁-C₃-alkoxy-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkoxycarbonylamino-, C₁-C₃-alkylcarbonyl-C₁-C₃-alkylamino-, C₁-C₃-alkoxycarbonyl-C₁-C₃-alkylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, cyano and nitro, wherein said heterocyclyls are five or six membered heterocyclyls containing from one to three heteroatoms each independently selected from the group consisting of oxygen, nitrogen and sulphur, and wherein the aryl or heterocyclyl components are unsubstituted or substituted by one to four substituents each independently selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, phenyl, cyano and nitro;
R⁷ and R⁸ are each independently selected from the group consisting of hydrogen, halogen, C₁-C₃-alkyl-, C₁-C₃-alkoxy-, C₁-C₃-haloalkyl- and C₁-C₃-haloalkoxy-;
R⁹ is selected from the group consisting of hydrogen, hydroxyl, halogen, C₁-C₆-alkyl, C₃-C₆-cycloalkyl, C₁-C₆-haloalkyl, C₂-C₆-haloalkenyl, C₁-C₆-alkoxy-, C₂-C₆-alkenyloxy-, C₃-C₆-cycloalkyl-C₁-C₃-alkyl-, C₁-C₆- alkoxy-C₁-C₃-alkyl-, C₁-C₆-alkoxy-C₂-C₆-alkoxy-, C₁-C₆-alkoxy-C₂-C₆-alkoxy-C₁-C₆-alkyl-, C₁-C₆-haloalkoxy-, C₁-C₆-haloalkoxy-C₁-C₃-alkyl-, C₁-C₆-alkyl-S(O)ₙ-, C₁-C₆-haloalkyl-S(O)ₙ-, aryl, aryl-S(O)ₙ-, heterocyclyl, heterocyclyl-S(O)ₙ-, aryloxy-, aryl-C₂-C₆-alkyl-, aryl-C₁-C₆-alkoxy-, heterocyclyloxy-, heterocyclyl-C₁-C₃-alkoxy-C₁-C₃-alkyl-, hydroxycarbonyl, hydroxycarbonyl-C₁-C₃-alkoxy-, C₁-C₃-alkoxycarbonyl-, C₁-C₃-alkoxycarbonyl-C₁-C₃-alkoxy-, C₁-C₃-alkylamino-, C₁-C₃-dialkylamino-, C₁-C₃-alkylamino-S(O)n-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-alkylaminocarbonyl-C₁-C₃-alkyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkoxycarbonylamino-, C₁-C₃-alkylcarbonyl-C₁-C₃-alkylamino-, C₁-C₃-alkoxycarbonyl-C₁-C₃-alkylamino-, C₁-C₃-alkyl-S(O)ₙ-amino-, C₁-C₃-alkyl-S(O)ₙ-C₁-C₃-alkylamino-, C₁-C₃-alkyl-S(O)ₙ-amino-C₁-C₃-alkyl-, cyano and nitro, wherein said heterocyclyls are five or six membered heterocyclyls containing from one to three heteroatoms each independently selected from the group consisting of oxygen, nitrogen and sulphur, and wherein the aryl or heterocyclyl components are unsubstituted or substituted by one to four substituents each independently selected from the group consisting of halo, C₁-C₃-alkyl, C₁-C₃-haloalkyl, C₁-C₃-alkoxy, C₁-C₃-haloalkoxy, C₁-C₆-alkyl-S(O)ₙ-, phenyl, cyano and nitro;
and wherein R⁵ and R⁹ can together form a saturated or unsaturated 5- or 6-membered carbocyclic or heterocyclic ring, said heterocyclic ring comprising one to three heteroatoms each independently selected from nitrogen, oxygen and sulphur, the 5- or 6-membered ring being unsubstituted or substituted by one to four R¹¹; or
R⁶ and R⁹ can together form a saturated or unsaturated 5- or 6-membered carbocyclic or heterocyclic ring, said heterocyclic ring comprising one to three heteroatoms each independently selected from nitrogen, oxygen and sulphur, the 5- or 6-membered ring being unsubstituted or substituted by one to four R¹¹; or
R⁶ and R⁸ can together form a saturated or unsaturated 5- or 6-membered carbocyclic or heterocyclic ring, said heterocyclic ring comprising one to three heteroatoms each independently selected from nitrogen, oxygen and sulphur, the 5- or 6-membered ring being unsubstituted or substituted by one to four R¹²;
R¹⁰ is selected from the group consisting of halo-, C₁-C₃-alkyl, C₁-C₃-haloalkyl and C₁-C₆-alkoxy;
R¹¹ is selected from the group of hydrogen, cyano, halo-, oxy-, C₁-C₃-alkyl-S(O)ₙ-, C₁-C₃-alkyl, C₂-C₃-alkenyl, C₂-C₃-alkynyl, C₁-C₃-alkoxy and C₁-C₃-haloalkyl;
R¹² is selected from the group of hydrogen, cyano, halo-, C₁-C₃-alkyl-S(O)ₙ-, C₁-C₃-alkyl, C₂-C₃-alkenyl, C₂-C₃-alkynyl, morpholinyl- and C₁-C₃-haloalkyl; and
n is 0, 1 or 2.

2. The compound according to claim 1 wherein R¹ and R² are each independently selected from the group consisting of C₁-C₆-alkyl, C₁-C₆-haloalkyl, C₃-C₆-cycloalkyl, C₁-C₆-alkoxy-C₁-C₃-alkyl-, C₃-C₆-cycloalkyl-C₁-C₃-alkyl- and oxetanyl.

3. The compound according to claim 2 wherein R¹ and R² are each independently selected from the group consisting of C₁-C₄-alkyl, cyclopropyl, oxetanyl, cyclopropylmethyl, difluoromethyl and trifluoroethyl.

4. The compound according to claim 3 wherein R¹ and R² are each independently selected from the group consisting of methyl, ethyl, isopropyl and cyclopropyl.

5. The compound according to any of claims 1 to 4 wherein R³ is selected from the group consisting of A1a, A1b, A1c, A1d, A1e, A1f, A1g, A1h, A1i, A1j, A2a, A2b and A2c as shown below 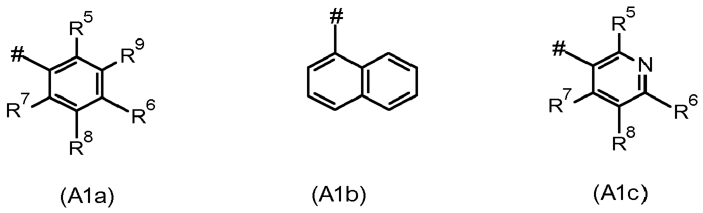 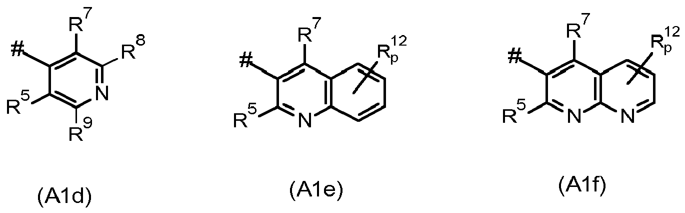 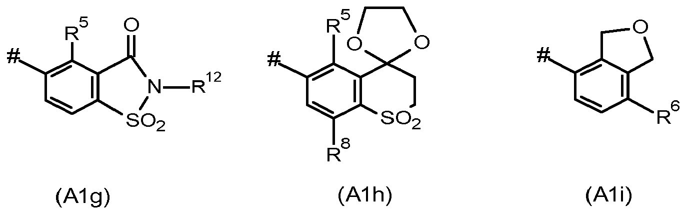 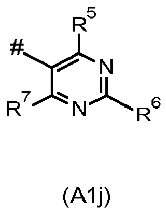 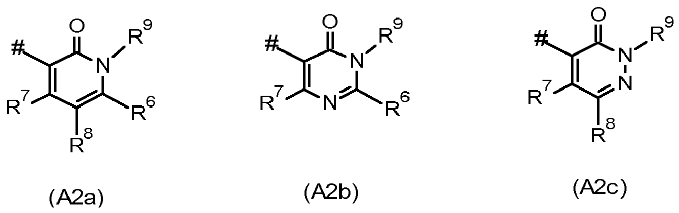 wherein #, R⁵, R⁶, R⁷, R⁸, R⁹ and R¹² are as previously defined and p is 0, 1, 2 or 3.

6. The compound according to claim 5 to wherein R³ is selected from the group consisting of A1a, A1c and A1d wherein #, R⁵, R⁶, R⁷, R⁸ and R⁹ are as previously defined.

7. The compound according to claim 6 wherein R³ is selected from the group consisting of phenyl, 3-pyridyl and 4-pyridyl.

8. The compound according to any of claims 1 to 7 wherein R⁵ is selected from the group consisting of hydroxyl, halogen, C₁-C₆-alkyl, C₃-C₆-cycloalkyl, C₁-C₆-haloalkyl, C₁-C₆-alkoxy, C₁-C₆-alkoxy-C₁-C₃-alkyl-, C₁-C₆-alkoxy-C₂-C₆-alkoxy-, C₁-C₆-alkoxy-C₂-C₆-alkoxy-C₁-C₃-alkyl-, C₁-C₆-haloalkoxy, C₁-C₆-haloalkoxy-C₁-C₃-alkyl-, C₁-C₆-alkyl-S(O)ₙ-, C₁-C₆-haloalkyl-S(O)ₙ-, heterocyclyl, heterocyclyl-C₁-C₃-alkoxy-C₁-C₃-alkyl-, C₁-C₃-alkylamino-, C₁-C₃-dialkylamino-, C₁-C₃-alkylamino-S(O)ₙ-, C₁-C₃-alkylamino-S(O)ₙ-C₁-C₃-alkyl-, C₁-C₃-dialkylamino-S(O)ₙ-, C₁-C₃-dialkylamino-S(O)ₙ-C₁-C₃-alkyl-, C₁-C₃-alkylaminocarbonyl-, C₁-C₃-dialkylaminocarbonyl-, C₁-C₃-dialkylaminocarbonyl-C₁-C₃-alkyl-, C₁-C₃-alkylcarbonylamino-, C₁-C₃-alkylcarbonyl-C₁-C₃-alkylamino-, C₁-C₃-alkyl-S(O)ₙ-amino-, C₁-C₃-alkyl-S(O)ₙ-C₁-C₃-alkylamino-, cyano and nitro, wherein said heterocyclyls are five or six membered heterocyclyls containing from one to three heteroatoms each independently selected from the group consisting of oxygen, nitrogen and sulphur, and wherein the heterocyclyl components may be unsubstituted or substituted by one to three substituents each independently selected from the group consisting of halo, C₁-C₃-alkyl, C₁-C₃-haloalkyl, C₁-C₃-alkoxy, C₁-C₃-haloalkoxy, cyano and nitro.

9. The compound according to claim 8 wherein R⁵ is selected from the group consisting of chloro, fluoro, methyl, trifluoromethyl, 2-fluoroethyl-, methoxyethoxymethyl-, trifluoromethoxymethyl-, methyl-S(O)ₙ-, ethyl-S(O)ₙ-, cyclopropyl, aryl, isoxazolinyl, morpholinyl, methyl-S(O)ₙ-dimethylamino-, cyano and nitro, wherein the heterocyclyl components may be unsubstituted or substituted by one to three substituents each independently selected from methyl and trifluoromethyl.

10. The compound according to claim 9 wherein R⁵ is selected from the group consisting of chloro, methyl, methyl-S(O)₂-, trifluoromethyl and cyclopropyl.

11. A composition comprising a herbicidally effective amount of at least one compound of the formula I or an agriculturally suitable salt or N-oxide thereof as defined in any of claims 1 to 10 and auxiliaries customary for formulating crop protection agents.

12. A method for controlling unwanted vegetation which comprises allowing a herbicidally effective amount of at least one compound of the formula I or of an agriculturally suitable salt or N-oxide thereof as defined in any of claims 1 to 10 to act on plants, their seed and/or their habitat.

13. Use of a compound of the formula I or of an agriculturally suitable salt or N-oxide thereof as defined in any of claims 1 to 10 for controlling unwanted vegetation.
